Europäisches Patentamt

European Patent Office

Office européen des brevets

(11) Publication number: **0 231 830**
A2

# (12) EUROPEAN PATENT APPLICATION

(21) Application number: 87100932.0

(22) Date of filing: 23.01.87

(51) Int. Cl.³: **G 01 N 33/543**
G 01 N 33/76
//G01N33/577

(30) Priority: 23.01.86 JP 12751/86

(43) Date of publication of application:
12.08.87 Bulletin 87/33

(84) Designated Contracting States:
AT BE CH DE ES FR GB GR IT LI LU NL SE

(71) Applicant: WAKO PURE CHEMICAL INDUSTRIES, LTD.
10, Doshomachi-3-chome
Higashi-ku Osaka(JP)

(72) Inventor: Sakata, Yoshitsugu c/o Osaka Research Lab.
Wako Pure Chem.Industries, Ltd. 6-1, Takada-cho
Amagasaki-shi Hyogo(JP)

(72) Inventor: Matsuura, Shuji c/o Osaka Research Lab.
Wako Pure Chem.Industries, Ltd. 6-1, Takada-cho
Amagasaki-shi Hyogo(JP)

(72) Inventor: Yamamoto, Tomoko c/o Osaka Research Lab.
Wako Pure Chem.Industries, Ltd. 6-1, Takada-cho
Amagasaki-shi Hyogo(JP)

(74) Representative: Hansen, Bernd, Dr.rer.nat. et al,
Hoffmann, Eitle & Partner Patentanwälte Arabellastrasse 4
D-8000 München 81(DE)

(54) Method of immunochemical assay.

(57) The present invention provides an extremely simple and effective method of a sandwich EIA (enzyme immunoassay) system, or more precisely, a method for the detection of an antigen or an antibody by means of a sandwich enzyme immunoassay system using a combined material of insoluble carrier/antigen/enzyme-labeled antibody or a combined material of insoluble carrier/antigen/enzyme-labeled anti-immunoglobulin-antibody which comprises an insoluble carrier having coated thereon a layer of an antibody or antigen specific to the antigen or antibody to be detected and a layer of an enzyme-labeled antibody or enzyme-labeled anto-immunoglobulin-antibody specific to the antigen or antibody to be detected. According to the method of the present invention, the immunoreaction product comprising fixed antibody/antigen to be detected/labeled antibody or fixed antigen/antibody to be detected/labeled antiimmunoglobulin-antibody can be formed on the surface of the insoluble carrier by only one incubation and only one washing operation.

Croydon Printing Company Ltd.

## METHOD OF IMMUNOCHEMICAL ASSAY

### FIELD OF THE INVENTION

The present invention relates to an improved method of a sandwich enzyme immunoassay system. More particularly, the present invention relates to a sandwich enzyme immunoassay system for the colorimetric determination of the immunoreaction product on an insoluble solid carrier.

### BACKGROUND OF THE INVENTION

Most physiologically active substances in living organisms have antigenicity, and the immunochemical binding reactions of these substances with the corresponding antibodies are extremely specific. Accordingly, a lot of efforts have heretofore been made to measure antigenic substances to be detected or antibodies specific thereto by utilzing the antigenantibody reaction, and various immunoassay methods have already been practiced. In particular, a radioimmunoassay (RIA) where an antigen or antibody is labeled with a radioisotope and an enzyme immunoassay (EIA) where an antigen or antibody is labeled with an enzyme are superior to any other methods as the sensitivity of detection is extremely higher and the quantitative accuracy is more excellent in these methods, and, therefore, these methods have widely been applied to the determination of various physiologically active substances.

The principle of determination is common to EIA and RIA, and the general methods include a competitive binding method and a sandwich method. For instance, a typical sandwich technique for the detection of an unknown antigen contained in a test sample (such as serums or urines) comprises binding an antibody for the detection of the unknown antigen onto the surface of a solid carrier and reacting the antibody with the antigen to be detected. After the surface of the solid carrier has been washed, a labeled antibody is reacted therewith and the amount of the antigen to be detected is calculated on the basis of the amount of the labeled antibody as bound. On the other hand, the competitive binding method comprises reacting an antibody fixed on the surface of a solid carrier with both an antigen to be detected and a labeled antigen simultaneously, and calculating the amount of the unknown antigen in the test specimen on the basis of the amount of the labeled antigen as bound on the surface of the carrier. A system for the detection of an unknown antibody in a test sample can be operated analogously. For instance, an antigen fixed on the surface of a solid carrier is reacted with a sample containing an antibody to be detected and then further reacted with a labeled anti-immunoglobulin antibody to form a sandwich complex comprising fixed antigen/antibody to be detected/labeled anti-immunoglobulin antibody, and, thus, the amount of the antibody to be detected can be calculated based on the calculated amount of the labeled antibody as bound on the surface of the solid carrier.

Labeling agents include radioisotopes, fluorescent substances, enzymes, etc. The methods using radioisotopes or fluorescent substances require special and exclusive equipments and instruments for detection. On the other hand, in the case of the method using enzymes as a labeling agent, a colorimetric determination can be used for the assay of the aimed substance by converting the substrate specific to the enzyme as a labeling agent into a colored substance.

The method for the detection of an antigen contained in a liquid sample using an enzyme-labeling sandwich colorimetric immunoassay system is described in, for example, Wada H.G. et al, Clin. Chem., 28, 1862-1866 (1982), etc. In general, the antibody specific to the antigen to be detected is used in two forms; in one form the antibody is bound to a water-insoluble carrier and in another it is labeled with a pertinent enzyme. In the first place, the fixed antibody bound to the carrier is reacted with an antigen to be detected, in a sample, and then, after the reaction mixture has been subjected to phase separation and washed, the antigen bound to the fixed antibody by the immunoreaction is reacted with the enzyme-labeled antibody. After the reaction mixture has again been subjected to phase separation, a substrate specific to the labeling enzyme is applied thereto for the enzyme reaction in either a solid phase or a liquid phase, and, thus, the amount of the antigen to be detected is determined.

In the liquid phase enzyme reaction, for example, the enzyme-labeled immunochemical reaction product formed on the water-insoluble carrier is first brought into contact with the substrate-containing solution, whereupon the coloration of the substrate occurs in the presence of the enzyme and a chemical reaction-terminating agent is added to terminate the reaction. Afterwards, the degree of the coloration of the soluble substrate in the solution is determined by the measurement of the absorbance thereof or with the naked eye. In this system, however, the enzyme reaction-terminating agent is required, and, further, in the measurement of the absorbance, an apparatus for the measurement is required. In addition, the permanent preservation of the test results is in fact impossible in the case of the reaction in the solution. Moreover, the labeled antibody liberated from the surface of the insoluble carrier in the substrate solution could cause fluctuation of the data. Thus, the liquid phase enzyme reaction suffers various problems.

On the other hand, in the solid phase enzyme reaction, for example, the immunochemical reaction product fixed on the water-insoluble carrier is brought into contact with a solution containing a soluble substrate specific to the labeling enzyme, whereby the substrate is converted into an insoluble colored substance and the resulting substance deposits on the solid phase. Afterwards, the solid phase is separated from the substrate-containing solution and is observed with the naked eye to thereby determine the amount of the antigen to be detected. According to this method, the enzyme reaction on the solid phase can easily be terminated by separating the solid phase itself from the substrate solution and washing the thus separated solid phase, and, therefore, a chemical reaction-terminating agent is not required. In addition, the permanent preservation of the colored carrier is possible as a record of the test results. The reaction of the undesirably liberated enzyme-labeled antibody does not have any influence on the enzyme reaction on the solid phase and, therefore, does not cause the fluctuation of the data.

The sandwich enzyme immunoassay system generally includes two methods for the formation of the immunochemical reaction products of the three parties, i.e., an antibody fixed on a carrier, an antigen, and an enzyme- labeled antibody. One of them is a two-step method comprising a first reaction in which the antibody fixed on the carrier is brought into contact with a liquid specimen containing the antigen to be detected and a second reaction in which the reaction product of the first reaction, after having been separated and washed, is further reacted with the enzyme-labeled antibody, the two incubation reaction steps being carried out in order. The other is a one-step method comprising one incubation step in which the enzyme-labeled antibody is previously added to the sample solution containing the antigen to be detected (EP-A-125118). The former two-step method is apparently complicated in the operation, since it involves two incubation steps and two separation and washing steps. On the other hand, the operation of the latter one-step method is easier than the former two-step method as far as the operation with respect to only the fixed antibody is concerned, since it involves one incubation and one separation and washing operations. However, the one-step method requires the operation for adding and blending the labeled antibody, which has been separately prepared, with the sample solution previously containing the antigen to be detected, and, therefore, it cannot be said that the one-step method is always a simple method which is to the satisfaction of the practitioners.

## SUMMARY OF THE INVENTION

An object of the present invention is to provide a simpler and more effective method of a sandwich enzyme immunoassay system wherein an immunoreaction product composed of fixed antibody/antigen to be detected/ labeled antibody or one composed of fixed antigen/antibody to be detected/labeled anti-immunoglobulin-antibody can be formed on an insoluble carrier in one incubation step.

Extensive investigations have been made on the sandwich enzyme immunoassay (EIA) system for the colorimetric determination of the immunoreaction product on an insoluble solid carrier in order to obtain a more simplified method in which the immunoreaction can be completed in one incubation operation. As a result, a new technique has been developed for the two-layer fixation in which an enzyme-labeled antibody layer is coated over the surface of an insoluble carrier having fixed thereon an antibody or antigen to form a two-layer-fixed constitution, and it has now been discovered that by using the two-layer-fixed enzyme immunoassay system, an immunoreaction product composed of the fixed antibody/antigen to be detected/labeled antibody or one composed of the fixed antigen/antibody to be detected/labeled anti-immunoglobulin-antibody can easily be formed on the insoluble carrier only by one operation of incubation with the specimen sample solution containing the antigen or antibody to be detected, and that the desired antigen or antibody can be detected by the colorimetric determination in which the insoluble carrier having thereon the immunoreaction product is brought into contact with a solution containing a substrate specific to the labeling enzyme, which may form an insoluble colored substance (insoluble dye) by reacting with the enzyme. The present invention has been achieved based on the discovery.

The present invention provides a method for the detection of an antigen or an antibody by a sandwich enzyme immunoassay system which comprises using a combined material of insoluble carrier/antibody/enzyme-labeled antibody or a combined material of insoluble carrier/antigen/enzyme-labeled anti-immunoglobulin-antibody which comprises an insoluble carrier having coated thereon a layer of an antibody or antigen specific to the antigen or antibody to be detected and a layer of an enzyme-labeled antibody or enzyme-labeled anti-immunoglobulin-antibody specific to the antigen or antibody to be detected, and preferably a combined material of insoluble carrier/antibody/enzyme-labeled antibody or a combined material of insoluble carrier/antigen/enzyme-labeled anti-immunoglobulin-antibody which is formed by chemically or physically binding and fixing an antibody or an antigen specific to the antigen or antibody to be detected on the surface of an insoluble carrier and then coating the surface of the fixed antibody or antigen with an enzyme-labeled antibody or an enzyme-labeled anti-immunoglobulin-antibody specific to the antigen or antibody to be detected to form a laminate layer.

DETAILED DESCRIPTION OF THE INVENTION

In the method of the present invention, two immunologically active reaction partners specific to the antigen or antibody to be measured (in which one of them is enzyme-labeled) are fixed on the same insoluble carrier, and, therefore, an immunoreaction product can be formed on the insoluble carrier by only one operation of incubation of the test sample containing the substance to be detected with the carrier. Afterwards, the substance to be detected can be detected by only one operation of phase separation, optionally with washing, followed by the enzyme reaction.

In the practice of the present invention, any conventional fixation means which is known in the field of EIA and RIA can be applied, without exception, to the fixation of the antibody or antigen specific to the substance to be detected, on the surface of the water-insoluble carrier. For instance, physical adsorption to the surface of the solid can be carried out, or to the surface of the solid can be carried out (Methods in Enzymology, 44, p148-169 (1976)), or alternatively, the fixation can be carried out by chemical coupling with a crosslinking agent by utilizing the functional groups such as an amino group, a carboxyl group, a hydroxyl group, a mercapto group or an amido group (Methods in Enzymology, 44, p11-148 (1976)). Regarding the insoluble carriers, any known carriers which are generally used in the field of EIA and RIA can be used in the method of the present invention. Suitable examples thereof include polymers such as polystyrene, polyvinyl chloride, polyethylene, polypropylene, modified cellulose and polyacrylamide and inorganic substances such as glass, silica and metal oxides, and preferably polystyrene and polypropylene. Preferred shapes of the carrier to be used in the present invention are, for example, spherical, rod-like, sheet-like or laminal (stick-like) forms, which, however, are not limitative.

According to the present invention, the layers of the two immunologically active reaction partners of fixed antibody and enzyme-labeled antibody or fixed antigen and enzyme-labeled anti-immunoglobulin antibody may be partly or entirely laminated one on the other or provided on separate regions of the surface of the insoluble carrier. When the layers are provided on separate regions they may be arranged in various patterns. For example, the surface of a rod-like carrier may be divided into two regions lengthwise or radially. They can also be fixed on an insoluble carrier using covalent coupling or physical adsorption. Preferably, the proportion of the enzyme to the antibody in enzyme-labeled antibody is about 1.5-2.5:1.

According to a preferred embodiment for the layer formation and fixation of the second layer on the first layer in the present invention, a thin coating film of the enzyme-labeled antibody containing a water-soluble layer-forming (or coating film-forming) agent is coated on the first layer to obtain a two-layer constitution. The water-soluble layer-forming (or coating film-forming) agent includes, for example, synthetic polymers such as polyvinyl alcohol, polyvinyl pyrrolidone, polyethylene glycol and carboxyvinyl polymers; polysaccharides such as dextran, soluble starch and sucrose; cellulose derivatives such as methyl cellulose, carboxymethyl cellulose, hydroxypropyl cellulose, hydroxyethyl cellulose and hydroxypropylmethyl cellulose; proteins such as water-soluble gelatin and casein; and glycerol, etc. Preferably, water soluble gelatin (30 to 50 wt%) and sucrose (40 to 95 wt%) are employed.

The enzyme-labeled antibody after being stabilized is dissolved in a solution containing the water-soluble layer-forming (or coating film-forming) agent in a concentration of about 10 to 50 µg/mℓ based on active antibody, and the resulting solution is coated to form a layer laminated over the first fixed layer by physical adsorption. The enzyme-labeled antibody layer containing the water-soluble layer-forming (or coating film-forming) agent thus laminated on the first fixed layer easily peels off therefrom in the solution containing the substance to be detected, and the enzyme-labeled antibody rapidly reacts with the antigen or antibody to be detected in the solution to form the immunoreaction product composed of the antigen to be detected/enzyme-labeled antibody or the antibody to be detected/enzyme-labeled anti-immunoglobulin-antibody, and thereafter the antigen or antibody to be detected is allowed to react immunochemically with the antibody or antigen fixed on the water-insoluble carrier or the first fixed layer, to form a desired immuno-complex sandwich composed of the fixed antibody/antigen to be detected/enzyme-labeled antibody or the fixed antigen/antibody to be detected/ enzyme-labeled anti-immunoglobulin-antibody.

Examples of enzymes which can be used in the present invention as a labeling enzyme include alkaline phosphatase, peroxidase, glucose-oxidase, galactosidase, etc., with alkaline phosphatase and peroxidase being preferred.

The substrates specific to the labeling enzyme to be used in the present invention for the detection of the enzyme reaction are preferably those capable of forming insoluble colored precipitates (insoluble dyes) on the surface of the solid carrier by the enzyme reaction. For instance, typical substrates are listed in the following Table 1, which, however, are not limitative.

## TABLE 1

| Substrate | Property of Product | |
|---|---|---|
| | Color Formed | Solubility in Water |
| Substrates for Horse Radish Peroxidase | | |
| Diaminobenzidine/$H_2O_2$ | Brown | Insoluble |
| 4-Chloro-1-naphthol/$H_2O_2$ | Blue | Insoluble |
| Substrates for Alkaline Phosphatase | | |
| Naphthol AS-MX Phosphate/Fast Blue BB | Blue | Insoluble |
| Naphthol AS-MX Phosphate/Fast Red TR Salt | Red | Insoluble |
| 2-Naphthyl Phosphate/Fast Blue B | Violet Brown | Insoluble |
| 5-Bromo-4-chloroindolyl Phosphate/Nitro Blue Tetrazolium | Blue | Insoluble |
| 5-Bromo-4-chloroindolyl Phosphate | Blue | Insoluble |
| Naphthol-AS-Phosphate/Fast Blue BB | Blue | Insoluble |
| Naphthol-AS-BI-Phosphate/Fast Red TR | Red | Insoluble |

14

In the present invention, the first antibody to be fixed on the insoluble carrier for the detection and measurement of the antigen may be the same as or different from the second enzyme-labeled antigen to be layered and coated over the first antibody. These antibodies may be a polyclonal antibody as isolated from an animal anti-serum or may be a monoclonal antibody produced by hybridoma cells obtained by cell fusion technique; and the antibodies must be able to recognize at least two or more different antigenic sites (epitopes) in the antigen to be detected, which is indispensable for the combination of the sandwich system with the antibodies.

In the case of the detection and measurement of an antibody by the method of the present invention, an enzyme-labeled anti-immunoglobulin-antibody is preferably used in place of the enzyme-labeled antibody.

Immunologically active substances which may be measured by the method of the present invention include, for example, hormones such as human chorionic gonadotropin (hCG), human luteinizing hormone (hLH), prolactin, insulin; cancer antigens such as carcinoembryonic antigen (CEA), α-fetoprotein (AFP); enzyme proteins such as γ-glutamyl transpeptidase (γ-GTP), amylase, alkaline phosphatase; anti-viral antibodies against clinically noticed viruses such as rubella virus, herpes virus, hepatitis virus, ATL virus, AIDS virus; substances capable of serving as a hapten, such as steroid hormones, e.g., cortisol, progesterone, etc., thyroid hormones, e.g., triiodothyronine, thyroxine, etc., and medicines, e.g., digoxin, morphine, nicotine, etc. These are, however, not whatsoever limitative but the method of the present invention can be applied to any immunologically active substances capable of being measured by utilizing the immunoreaction for the measurement thereof.

Now, the present invention will be explained in greater detail with reference to the following examples, which, however, are not intended to be interpreted as limiting the scope of the present invention.

EXAMPLE   1

Preparation of Insoluble Carrier Having Both Fixed Antibody and Enzyme-Labeled Antibody:

One end part (or the part to which the antibody is to be adsorbed, having a size of 8 × 15 mm) of a polypropylene stick (8.0 × 87 × 3 mm) formed by extrusion molding as an insoluble carrier was dipped in 10 mM of phosphate buffer solution (PBS) (pH 7.3) containing 1 mg/mℓ of hCG-specific monoclonal antibody and 0.8 w/v% of NaCℓ for 2 hours at 37°C to fix the anti-hCG-monoclonal antibody on the insoluble carrier stick. The antibody used was obtained by hybridization of spleen lymphocytes of an hCG-immunized mouse and mouse myeloma cells (NS-1) followed by cloning of the resulting hybridoma cells in a known manner (Kohler and Milstein, Nature, 256, 495-499 (1975)).

The excessive antibody was removed by washing with PBS containing 0.05% of Tween 20, and then the stick was dipped in PBS containing 3% sucrose and 1% BSA (bovine serum albumin) for 2 hours at room temperature, and then this was taken out therefrom and air-dried.

Apart from this stick, another coated stick was prepared as follows. A polyclonal antibody was produced by immunizing a rabbit according to the method described in S. Matsuura, H.C. Chen and G.D. Hodgen, Biochemistry, 17, 575-580, 1978, with a synthetic peptide corresponding to the carboxyl terminal part of ß-subunit of hCG (or corresponding to the 111th to 145th site of the structural amino acid sequence) obtained by a solid phase peptide synthesis method (G. Barany and R.B. Merrifield, The Peptides, 2, 1-284, 1980), and the polyclonal antibody was fixed on a stick in the same manner as above to obtain another antibody-fixed carrier. On the other hand, a monoclonal antibody which is specific to the ß-subunit of hCG was produced according to the method of Kohler and Milstein as described above, and then the resulting antibody was conjugated with calf intestinal alkaline phosphatase according to a known method (E. Engvall, Methods in Enzymology, 70, 419-483, 1983) to obtain the enzyme-labeled antibody. The thus labeled antibody had a ratio of enzyme to antibody of about 2:1. The labeled antibody was dissolved in 10 mM PBS containing 80% sucrose and 1% BSA in a concentration of about 30 µg/mℓ based on the amount of the active antibody.

The antibody-carried part of the stick, on which the monoclonal antibody or polyclonal antibody had previously been fixed, was dipped in this solution for 2 hours at 37°C, and then the stick was taken out from the solution and air-dried. Thus, the insoluble carrier stick having both the fixed antibody and the enzyme-labeled antibody, where the enzyme-labeled antibody was layered and coated over the first fixed antibody, was obtained.

### EXAMPLE 2

Measurement of hCG by Sandwich Enzyme Immunoassay System:

Then, the stick was taken out from the urine sample and fully washed with distilled water by rotating 1,000 mIU/mℓ of hCG in such state that the top end part of the stick where the antibodies were layered and fixed was completely dipped therein, and kept at room temperature for 15 minutes.

18

Then, the stick was taken out from the urine sample and fully washed with distilled water by rotating the stick in a beaker containing distilled water. After excess water was ligtly absorbed by putting the stick on a filter paper the stick was put in 1 mℓ of a substrate solution. The substrate solution used was obtained by dissolving 0.1 mg/mℓ of NPMX (3-hydroxy-2-naphtho-2',4'-xylidine dihydrogen phosphate) and 0.6 mg/mℓ of Fast Red TR (5-chloro-2-toluenediazonium chloride) in 0.1 M tris-HCℓ buffer (pH 7.4) containing 0.9% NaCℓ, 1 mM MgCℓ$_2$ and 0.1% NaN$_3$. The reaction was carried out for 10 minutes at room temperature, and the stick was taken out from the substrate solution and the water was absorbed with a blotting paper and then the resulting stick was air-dried.

The amount of hCG contained in each urine sample was shown on the stick by pink coloration the intensity of which was in proportion to the amount of hCG in the urine sample. The stick dipped in the urine sample containing no hCG did not color but remained colorless. Using the system comprising the anti-hCG-monoclonal antibody as the first antibody or using the system comprising the anti-hCG-ß-carboxyl terminal peptide-polyclonal antibody, hCG could be detected in the same manner.

The same detection was carried out on a variety of urine samples each containing 0 to 1,000 mIU/mℓ of hLH by utilizing the above-mentioned measurement method. As a result, pink coloration was observed in the stick comprising the anti-hCG-monoclonal antibody as the first antibody the intensity of which was in proportion to the amount of the hLH. Thus, it was confirmed that this stick was usable for the detection of not only hCG but also hLH.

On the other hand, when the stick having the anti-hCG-β-carboxyl terminal peptide-polyclonal antibody as the first antibody was used, the coloration resulting from the existence of hLH did not occur, and, therefore, the specific detection of hCG was possible.

Semi-quantitative determination of the content of hCG in specimens is possible by comparing the coloration of the specimen with that of the above-mentioned standard hCG sample with the naked eye, and, thus, the diagnosis of pregnancy is possible by the qualitative observation of the specimen to be detected with the naked eye in accordance with the measurement method of the present invention.

While the invention has been described in detail and with reference to specific embodiments thereof, it will be apparent to one skilled in the art that various changes and modifications can be made
therein without departing from the spirit and scope
thereof.

CLAIMS:

1. A method for the detection of an antigen or an antibody by a sandwich enzyme immunoassay system, which comprises using a combined material of insoluble carrier/antibody/enzyme-labeled antibody or a combined material of insoluble carrier/antigen/enzyme-labeled anti-immunoglobulin-antibody which comprises an insoluble carrier having coated thereon a layer of an antibody or antigen specific to the antigen or antibody to be detected and a layer of an enzyme-labeled antibody or enzyme-labeled anti-immunoglobulin-antibody specific to the antigen or antibody to be detected.

2. A method for the detection of an antigen or an antibody by a sandwich enzyme immunoassay system as claimed in Claim 1, wherein said combined material of insoluble carrier/antibody/enzyme-labeled antibody or combined material of insoluble carrier/antigen/enzyme-labeled anti-immunoglobulin-antibody is one formed by chemically or physically binding and fixing an antibody or an antigen specific to the antigen or antibody to be detected onto the surface of an insoluble carrier and then coating the surface of the fixed antibody or antigen with enzyme-labeled antibody or an enzyme-labeled anti-immunoglobulin-antibody specific to the antigen or antibody to be detected to form a laminate layer.

3. A method for the detection of an antigen or an antibody by a sandwich enzyme immunoassay system as claimed in Claim 2, wherein the combined material of insoluble carrier/antibody/enzyme-labeled antibody or the combined material of insoluble carrier/antigen/enzyme-labeled anti-immunoglobulin-antibody formed by coating the surface of the fixed antibody or antigen with an enzyme-labeled antibody or an enzyme-labeled anti-immunoglobulin-antibody specific to the antigen or antibody to be detected is incubated in a sample solution containing the antigen or antibody to be detected to thereby form an immunoreaction product composed of the fixed antibody/antigen to be detected/ labeled antibody or one composed of the fixed antigen/ antibody to be detected/labeled anti-immunoglobulin-antibody on the insoluble carrier, and then the resulting product is brought into contact with a solution containing a substrate to the labeling enzyme, which can form an insoluble colored substance precipitate on the surface of the solid carrier by the enzyme reaction, to thereby form the insoluble enzyme reaction product precipitate (insoluble colored substance precipitate) on the solid carrier, and the coloration of said precipitate is measured with the naked eye or by the use of a measuring apparatus.

4. A method for the detection of an antigen or an antibody by a sandwich enzyme immunoassay system as claimed in Claim 3, wherein a water-soluble layer-forming (or coating film-forming) agent is used in the formation of the coat layer of the enzyme-labeled antibody or the enzyme-labeled anti-immunoglobulin-antibody on the fixed antibody or antigen.

5. A method for the detection of an antigen or an antibody by a sandwich enzyme immunoassay system as claimed in Claim 4, wherein the water-soluble layer-forming (or coating film-forming) agent is selected from the group consisting of synthetic polymers, polysaccharides, cellulose derivatives, proteins and glycerol.

6. A method for the detection of an antigen or an antibody by a sandwich enzyme immunoassay system as claimed in Claim 5, wherein the insoluble carrier is selected from the group consisting of polystyrene, polyvinyl chloride, polyethylene, polypropylene, modified cellulose, polyacrylamide, glass, silica and metal oxides.

7. A method for the detection of an antigen or an antibody by a sandwich enzyme immunoassay system as claimed in Claim 6, wherein the insoluble carrier is a stick-like or a rod-like carrier.

8. A method for the detection of an antigen or an antibody by a sandwich enzyme immunoassay system as claimed in Claim 1, wherein said combined material of insoluble carrier/antibody/enzyme-labeled antibody or combined material of insoluble carrier/antigen/enzyme-labeled anti-immunoglobulin-antibody is one formed by chemically or physically binding and fixing (a) an antibody or an antigen specific to the antigen or antibody to be detected and (b) an enzyme-labeled antibody or an enzyme-labeled anti-immunoglobulin-antibody specific to the antigen or antibody to be detected onto different regions of the surface of an insoluble carrier which regions do not overlap each other or partly overlap each other.

9. A method for the detection of an antigen or an antibody by a sandwich enzyme immunoassay system as claimed in Claim 8, wherein the combined material of insoluble carrier/antibody/enzyme-labeled antibody or the combined material of insoluble carrier/antigen/ enzyme-labeled anti-immunoglobulin-antibody is incubated in a sample solution containing the antigen or antibody to be detected to thereby form an immunoreaction product. composed of the fixed antibody/antigen to be detected/ labeled antibody or one composed of the fixed antigen/ antibody to be detected/labeled anti-immunoglobulin-antibody on the insoluble carrier, and then the resulting product is brought into contact with a solution containing a substrate to the labeling enzyme, which can form an insoluble colored substance precipitate on the surface of the solid carrier by the enzyme reaction, to thereby form the insoluble enzyme reaction product precipitate (insoluble colored substance precipitate) on the solid carrier, and the coloration of said precipitate is measured with the naked eye or by the use of a measuring apparatus.

10. A method for the detection of an antigen or an antibody by a sandwich enzyme immunoassay system as claimed in Claim 9, wherein the insoluble carrier is selected from the group consisting of polystyrene, polyvinyl chloride, polyethylene, polypropylene, modified cellulose, polyacrylamide, glass, silica and metal oxides.

11. A method for the detection of an antigen or an antibody by a sandwich enzyme immunoassay system as claimed in Claim 10, wherein the insoluble carrier is a stick-like or a rod-like carrier.

0231830

12. A method for the detection of an antigen or an antibody by a sandwich enzyme immunoassay system as claimed in Claim 9, wherein said different regions do not overlap each other.

13. A method for the detection of an antigen or an antibody by a sandwich enzyme immunoassay system as claimed in Claim 9, wherein said different regions partly overlap each other.

14. A method for the detection of an antigen or an antibody by a sandwich enzyme immunoassay system as claimed in Claim 13, wherein a water-soluble layer-forming (or coating film-forming) agent is used in the formation of the coat layer of the enzyme-labeled antibody or the enzyme-labeled anti-immunoglobulin-antibody on the fixed antibody or antigen.

15. A method for the detection of an antigen or an antibody by a sandwich enzyme immunoassay system as claimed in Claim 14, wherein the water-soluble layer-forming (or coating film-forming) agent is selected from the group consisting of synthetic polymers, polysaccharides, cellulose derivatives proteins and glycerol.

16. A stick-like or rod-like carrier for the detection of an antigen or an antibody by a sandwich enzyme immunoassay system, which has coated thereon a layer of an antibody or antigen specific to the antigen or antibody to be detected and a layer of an enzyme-labeled antibody or enzyme-labeled anti-immunoglobulin-antibody specific to the antigen or antibody to be detected.

17. A stick-like or rod-like carrier for the detection of an antigen or an antibody by a sandwich enzyme immunoassay system as claimed in Claim 16, wherein said layer of the antibody or antigen specific to the antigen or antibody to be detected is fixed onto the surface of the carrier by chemical or physical binding and said layer of the enzyme-labeled antibody or enzyme-labeled anti-immunoglobulin-antibody specific to the antigen or antibody to be detected is formed by coating the surface of the fixed antibody or antigen with the enzyme-labeled antibody or enzyme-labeled anti-immunoglobulin-antibody to form a laminate layer.

18. A stick-like or rod-like carrier for the detection of an antigen or an antibody by a sandwich enzyme immunoassay system as claimed in Claim 17, wherein said layer of the enzyme-labeled antibody or the enzyme-labeled anti-immunoglobulin-antibody contains a water-soluble layer-forming (or coating film-forming) agent.

19. A stick-like or rod-like carrier for the detection of an antigen or an antibody by a sandwich enzyme immunoassay system as claimed in Claim 18, wherein the water-soluble layer-forming (or coating film-forming) agent is selected from the group consisting of synthetic polymers, polysaccharides, cellulose derivatives, proteins and glycerol.

20. A stick-like or rod-like carrier for the detection of an antigen or an antibody by a sandwich enzyme immunoassay system as claimed in Claim 19, wherein the stick-like or rod-like carrier is selected from the group consisting of polystyrene, polyvinyl chloride, polyethylene, polypropylene, modified cellulose, polyacrylamide, glass, silica and metal oxides.

21. A stick-like or rod-like carrier for the detection of an antigen or an antibody by a sandwich enzyme immunoassay system as claimed in Claim 20, wherein the stick-like or rod-like carrier is selected from the group consisting of polystylene and polypropylene.

22. A stick-like or rod-like carrier for the detection fo an antigen or antibody by a sandwich enzyme immunoassay system as claimed in Claim 16, said layer of the antibody or antigen specific to the antigen or antibody to be detected and said layer of the enzyme-labeled antibody or an enzyme-labeled anti-immunoglobulin-antibody specific to the antigen or antibody to be detected are fixed by chemical or physical binding onto different regions of the surface of the carrier which regions do not overlap each other or partly overlap each other.

23. A stick-like or rod-like carrier for the detection of an antigen or an antibody by a sandwich enzyme immunoassay system as claimed in Claim 22, wherein the stick-like or rod-like carrier is selected from the group consisting of polystyrene, polyvinyl chloride, polyethylene, polypropylene, modified cellulose, polyacrylamide, glass, silica and metal oxides.

24. A stick-like or rod-like carrier for the detection of an antigen or an antibody by a sandwich enzyme immunoassay system as claimed in Claim 23, wherein the stick-like or rod-like carrier is selected from the group consisting of polystylene and polypropylene.

25. A stick-like or rod-like carrier for the detection of an antigen or an antibody by a sandwich enzyme immunoassay system as claimed in Claim 22, wherein said different regions do not overlap each other.

26. A stick-like or rod-like carrier for the detection of an antigen or an antibody by a sandwich enzyme immunoassay system as claimed in Claim 22, wherein said different regions partly overlap each other.

27. A stick-like or rod-like carrier for the detection of an antigen or an antibody by a sandwich enzyme immunoassay system as claimed in Claim 26, wherein said layer of the enzyme-labeled antibody or the enzyme-labeled anti-immunoglobulin-antibody contains a water-soluble layer-forming (or coating film-forming) agent.

28. A stick-like or rod-like carrier for the detection of an antigen or an antibody by a sandwich enzyme immunoassay system as claimed in Claim 27, wherein the water-soluble layer-forming (or coating film-forming) agent is selected from the group consisting of synthetic polymers, polysaccharides, cellulose derivatives, proteins and glycerol.